# FASCICULE DE BREVET EUROPEEN

(11) **EP 4 154 262 B1**
(45) Date de publication et mention de la délivrance du brevet: **01.07.2026**
(21) Numéro de dépôt: 21733487.9
(22) Date de dépôt: 20.05.2021
(51) Int. Cl.: G16H 30/40

(54) **MÉTHODE DE PRÉDICTION DE LA RÉCIDIVE D'UNE LÉSION PAR ANALYSE D'IMAGES**
VERFAHREN ZUR VORHERSAGE DES WIEDERAUFTRETENS EINER LÄSION DURCH BILDANALYSE
METHOD FOR PREDICTING THE RECURRENCE OF A LESION BY IMAGE ANALYSIS

(30) Priorité: 20.05.2020 FR 2005338
(43) Date de publication de la demande: 29.03.2023
(73) Titulaire: Quantum Surgical, 34000 Montpellier (FR)
(72) Inventeur: OUBEL, Estanislao, 34090 MONTPELLIER (FR); BLONDEL, Lucien, 34070 MONTPELLIER (FR); NAHUM, Bertin, 34170 CASTELNAU-LE-LEZ (FR); BADANO, Fernand, 69006 LYON (FR)
(74) Mandataire: Santarelli
(86) Numéro de dépôt international: PCT/FR2021/050906
(87) Numéro de publication internationale: WO 2021/234304

(56) Documents cités:
- EGGER JAN ET AL: "Interactive Volumetry Of Liver Ablation Zones", vol. 5, no. 1, 20 October 2015 (2015-10-20), XP055772148, Retrieved from the Internet <URL:http://www.nature.com/articles/srep15373> [retrieved on 20210329], DOI: 10.1038/srep15373
- ZHANG SIYUAN ET AL: "Detection and Monitoring of Thermal Lesions Induced by Microwave Ablation Using Ultrasound Imaging and Convolutional Neural Networks", IEEE JOURNAL OF BIOMEDICAL AND HEALTH INFORMATICS, IEEE, PISCATAWAY, NJ, USA, vol. 24, no. 4, 5 September 2019 (2019-09-05), pages 965 - 973, XP011781554, ISSN: 2168-2194, [retrieved on 20200402], DOI: 10.1109/JBHI.2019.2939810

## Description

### DOMAINE TECHNIQUE DE L'INVENTION

Le domaine de l'invention est celui de l'évaluation d'une intervention médicale.

Plus précisément, l'invention concerne une méthode d'évaluation post-traitement d'un risque de récidive d'une liaison ablatée.

L'invention trouve notamment des applications pour évaluer une intervention médicale mini-invasive en prédisant un risque de récidive d'une lésion, telle qu'une tumeur ou une métastase. Une telle intervention médicale mini-invasive correspond par exemple à une ablation percutanée de lésion, par exemple une tumeur dans le foie, un poumon, un rein ou tout autre organe. Une ablation percutanée consiste généralement à guider par l'image l'insertion d'une ou plusieurs aiguilles à travers la peau pour atteindre et détruire une lésion.

### ÉTAT DE LA TECHNIQUE

Il est connu de l'art antérieur des techniques permettant d'évaluer l'efficacité d'une intervention et de prédire un risque de récidive d'une lésion.

Une telle technique consiste par exemple suite à l'ablation d'une lésion, à déterminer dans quelle mesure la région d'ablation recouvre la lésion. En comparant le volume de la région d'ablation au volume de la lésion, il est possible de déterminer les marges d'ablation. En pratique, il est généralement recommandé d'avoir des marges d'ablation d'au moins cinq millimètres.

Afin de déterminer ces marges, le volume de la lésion est généralement déterminé lors de la planification de l'intervention et comparé au volume de la région d'ablation qui est segmenté par un opérateur sur au moins une image post-opératoire.

L'inconvénient majeur est que le volume de la région d'ablation est généralement déterminé avec peu de précision, d'une manière dépendant souvent de l'opérateur ayant effectué la segmentation. En outre, la qualité des images post-opératoires est souvent mauvaise, ce qui contribue à introduire des incertitudes dans la segmentation. Par conséquent, une corrélation entre des marges d'ablation et un risque de récidive d'une lésion est difficile à établir.

Afin d'améliorer les techniques de l'art antérieur, il est connu d'utiliser des méthodes de segmentation automatique de la région d'ablation.

Une telle technique est par exemple décrite dans la publication scientifique de Zhang et al, intitulée « Detection and Monitoring of Thermal Lesions Induced by Microwave Ablation Using Ultrasound Imaging and Convolutional Neural Networks », paru en septembre 2019. La méthode de segmentation décrite dans cette publication permet de calculer les marges de la région d'ablation en segmentant une image échographique pré-opératoire et une image échographique post-opératoire.

Toutefois, la méthode de segmentation décrite dans cette publication ne permet pas d'effectuer de prédiction du risque de récidive car la précision de la segmentation automatique de la région d'ablation est faible. D'une part la méthode de segmentation est limitée à une matrice de sous-échantillonnage d'une image de la région d'ablation, dont la taille est fixe, généralement égale à 4 mm², limitant ainsi l'utilisation de la méthode à des régions de dimensions réduites. En outre, il est nécessaire de connaître la position de la région d'ablation pour fixer la position de la matrice de sous-échantillonnage, rendant la méthode difficile à utiliser en l'absence de points de référence constants à l'intérieur de la matrice de sous-échantillonnage dans les images pré-opératoire et post-opératoire. Enfin, en raison de la nature et de la qualité des images échographiques en deux dimensions, l'anatomie d'intérêt peut être difficile à cerner, rendant la segmentation de la région peu précise, aboutissant notamment à des observations erronées où la région d'ablation telle que segmentée n'englobe pas la lésion objet de l'ablation. La publication "Interactive Volumetry Of Liver Ablation Zones", Egger Jan et al.,vol. 5, no. 1, 20 octobre 2015 (2015-10-20), XP055772148,DOI: 10.1038/srep15373 divulgue une méthode d'analyse d'une tumeur récurrente. Elle comprend un suivi dans le temps de l'altération des tissus autour d'une région d'ablation d'une tumeur afin de détecter des récidives.

En outre, les méthodes de segmentation automatique donnent des résultats cohérents pour des régions homogènes, c'est-à-dire par exemple pour un os, des vaisseaux sanguins, ou une lésion, ou lorsque l'image comprend un nombre connu de régions. Dans le cas de région d'ablations, les résultats de segmentation obtenus sont peu cohérents car les régions d'ablation sont des régions très complexes composées généralement de différentes matières telles que du gaz, des cellules nécrosées, des cellules saines, du produit de contraste résiduel, de la calcification, etc. Par ailleurs, la segmentation est généralement effectuée sur des images médicales généralement floues et peu contrastées, rendant la segmentation automatique de l'image difficile.

Aucun des systèmes actuels ne permet de répondre simultanément à tous les besoins requis, à savoir de proposer une technique permettant d'évaluer finement un risque de récidive d'une lésion préalablement ablatée, notamment à partir d'une image médicale peu nette et/ou peu contrastée, sans nécessiter l'intervention d'un opérateur expérimenté.

### EXPOSÉ DE L'INVENTION

La présente invention vise à remédier à tout ou partie des inconvénients de l'état de la technique cités ci-dessus.

À cet effet, l'invention vise un procédé d'évaluation post-traitement d'une ablation d'une partie d'une anatomie d'intérêt d'un individu, l'anatomie d'intérêt comprenant au moins une lésion, la partie de l'anatomie d'intérêt ablatée étant appelée région d'ablation.

L'ablation est de type percutanée ou mini-invasive, ce qui consiste généralement à l'insertion d'au moins une aiguille à travers la peau pour atteindre et détruire une lésion. Plusieurs techniques d'ablation sont possibles : radiofréquence, micro-ondes, électroporation, laser, cryothérapie, ultrasons, etc.

L'anatomie d'intérêt peut être un foie, un poumon, un rein ou tout autre organe susceptible d'avoir une lésion.

Selon l'invention, le procédé d'évaluation post-traitement comprend des étapes de :
- acquisition d'une image médicale post-opératoire de l'anatomie d'intérêt de l'individu ;
- recalage de l'image post-opératoire et d'une image médicale de l'anatomie d'intérêt de l'individu, acquise avant le traitement chirurgical, dite image médicale pré-opératoire, l'image médicale pré-opératoire et l'image médicale post-opératoire recalées formant un couple d'images médicales de l'anatomie d'intérêt de l'individu ;
- évaluation d'un risque de récidive de la lésion de l'anatomie d'intérêt de l'individu par une méthode d'apprentissage automatique, de type réseau de neurones, analysant le couple d'images médicales de l'anatomie d'intérêt de l'individu, ladite méthode d'apprentissage automatique étant préalablement entraînée lors d'une phase dite d'entraînement sur une base de données comportant une pluralité de couples d'images médicales d'une anatomie d'intérêt identique d'un ensemble de patients, chaque couple d'images médicales de la base de données étant associé à un statut de récidive d'une lésion de l'anatomie d'intérêt dudit patient.

Ainsi, un risque de récidive d'une lésion est prédit à partir de données cliniques précédentes en effectuant une analyse à très grande échelle de couples d'images médicales d'une même anatomie, par exemple un foie lorsque l'anatomie d'intérêt objet du traitement est un foie. Cette analyse permet d'avoir une estimation plus fine du risque de récidive de la lésion objet de l'ablation, sans nécessiter la présence d'un opérateur expérimenté. Le procédé d'évaluation post-traitement selon l'invention offre ainsi au personnel médical une meilleure vision du traitement appliqué à l'individu, en lui permettant d'évaluer la nécessité d'un traitement complémentaire si le risque de récidive s'avère important.

Le statut de récidive prend généralement soit une valeur dite positive lorsqu'une récidive a été constatée soit une valeur dite négative où aucune récidive n'a été constatée à une date de contrôle post-opératoire.

Le risque de récidive prend quant à lui généralement la forme d'une probabilité comprise entre 0 et 1.

Avantageusement, le risque de récidive est évalué à une échéance prédéterminée suite au traitement, chaque couple d'images médicales de la base de données étant également associé à une date de récidive lorsque le statut de récidive est positif.

L'échéance peut être par exemple à 1 mois, à 3 mois, à 6 mois, à 1 an, à 2 ans, à 5 ans, voire à 10 ans suite au traitement par ablation.

On entend par image médicale pré-opératoire une image médicale acquise avant le traitement par ablation et par image médicale post-opératoire une image médicale acquise après le traitement par ablation.

Dans des modes particuliers de mise en œuvre de l'invention, tout ou partie des couples d'images d'entrainement de la base de données sont recadrés, après recalage, autour de la région d'ablation comprenant au moins une lésion, le recadrage des images étant effectué selon un cadre commun de dimension prédéterminée, l'ensemble des centres de la région d'ablation des couples d'images recadrés formant une constellation de points distincts à l'intérieur du cadre commun.

Ainsi, en répartissant la position des régions d'ablation dans le cadre commun, il est possible de réduire les erreurs de prédiction de la méthode d'apprentissage automatique. Dans le cas où l'ensemble des régions d'ablation seraient dans la même position dans le cadre commun, la méthode d'apprentissage automatique considérerait principalement les couples d'images ayant une région d'ablation dans cette position particulière, entraînant ainsi des erreurs de prédiction dans le cas où la région d'ablation serait dans une autre position.

Dans des modes particuliers de mise en œuvre de l'invention, pour l'ensemble des couples d'images médicales de la base de données, préalablement recadrées, la partie du corps de l'individu comprise dans l'image est divisée en une pluralité d'unités élémentaires de taille unique, le nombre d'unités élémentaires étant partagé en deux parts quasiment égales entre la partie du corps humain délimitée par la région d'ablation et le reste de la partie du corps de l'individu comprise dans l'image.

En d'autres termes, le nombre d'unités élémentaires est partagé en deux parts quasiment égales entre la partie du corps humain délimitée par la région d'ablation et le reste de la partie du corps de l'individu comprise dans les couples d'images recadrés de la base de données.

Il convient de souligner que la répartition équivalente entre les unités élémentaires correspondant à une région d'ablation et les unités élémentaires à une région de non ablation peut être analysée au niveau d'une image ou globalement au niveau de l'ensemble des images.

Les unités élémentaires sont généralement appelées pixels dans le cadre d'images bidimensionnelles ou voxels dans le cadre d'images tridimensionnelles.

On entendra par parts quasiment égales lorsque les deux ensembles d'unités élémentaires sont constitués du même nombre d'unités élémentaires ou lorsque la différence de nombre d'unités élémentaires de chacun des deux ensembles est par exemple inférieure à 5 % du nombre d'unités élémentaires des deux ensembles.

Dans des modes particuliers de mise en œuvre de l'invention, la base de données de couples d'images médicales comprend au moins un couple d'images dépourvues de région d'ablation.

Ainsi, la méthode d'apprentissage automatique est mieux entraînée en ayant au moins un couple d'images où aucune ablation n'a été effectuée.

Dans des modes particuliers de mise en œuvre de l'invention, le procédé d'évaluation post-traitement comprend également une étape de détermination d'un masque d'ablation complémentaire lorsque le risque de récidive est supérieur à une valeur seuil prédéterminée.

Ainsi, une proposition de traitement est estimée dans un but d'améliorer le risque de récidive par le procédé d'évaluation post-traitement. Il convient de souligner que cette proposition de traitement complémentaire est non contraignante, pouvant être suivie ou non par le personnel médical.

Il convient de souligner que le masque d'ablation complémentaire est une visualisation de la région à ablater lors du traitement complémentaire. Le masque d'ablation est généralement généré, lorsque le risque de récidive est supérieur à une valeur seuil, par exemple 0,5, après avoir :
- détecté et segmenté la lésion et la région d'ablation respectivement dans l'image pré-opératoire et dans l'image post-opératoire ;
- déterminer au moins une position de la récidive.

En outre, la détection et la segmentation peuvent avoir été effectuées automatiquement ou manuellement par un opérateur.

Dans des modes particuliers de mise en œuvre de l'invention, l'étape de détermination d'un masque d'ablation complémentaire comprend une sous-étape de segmentation de la région d'ablation dans l'image post-opératoire de l'anatomie d'intérêt de l'individu.

La segmentation peut être effectuée automatiquement ou de manière manuelle par un opérateur. Il convient de souligner que la région d'ablation a généralement été préalablement détectée dans l'image où au moins un opérateur a indiqué sa position dans l'image.

Dans des modes particuliers de mise en œuvre de l'invention, l'étape de détermination d'un masque d'ablation complémentaire comprend une sous-étape de détection de la région d'ablation dans l'image post-opératoire de l'anatomie d'intérêt de l'individu.

Dans des modes particuliers de mise en œuvre de l'invention, l'étape de détermination d'un masque d'ablation complémentaire comprend une sous-étape de détection de la lésion dans l'image pré-opératoire de l'anatomie d'intérêt de l'individu.

Ainsi, la segmentation peut être effectuée autour de la lésion détectée dans l'image médicale.

Dans des modes particuliers de mise en œuvre de l'invention, l'étape de détermination d'un masque d'ablation complémentaire comprend une sous-étape de détermination de la position de la récidive en fonction d'au moins un prédicteur de risque choisi parmi la liste comprenant :
- une marge d'ablation entre la région d'ablation et la liaison,
- une distance entre un centre de masse de la lésion et un centre de masse de la région d'ablation,
- la régularité et de la netteté des bords de la région d'ablation par rapport au tissu sain environnant,
- le rapport entre le volume de la lésion et le volume de la région d'ablation,
- une position de la lésion par rapport au centre de l'anatomie d'intérêt.

Dans des modes particuliers de mise en œuvre de l'invention, les images médicales sont des images tridimensionnelles.

Il convient de souligner qu'une image tridimensionnelle peut par exemple correspondre à une collection d'images bidimensionnelles effectuées à intervalles généralement réguliers le long d'un axe prédéfini.

Dans des modes particuliers de mise en œuvre de l'invention, chaque image pré-opératoire est acquise selon une première technique d'acquisition d'image et chaque image post-opératoire est acquise selon une deuxième technique d'acquisition d'image, la première technique et la deuxième technique étant identiques ou distinctes.

Dans des modes particuliers de mise en œuvre de l'invention, le procédé d'évaluation post-traitement comprend également une étape de proposition d'une trajectoire d'un instrument médical jusqu'à un point cible d'une région d'ablation définie par le masque d'ablation complémentaire.

Il convient de souligner que cette étape de proposition d'une trajectoire, pouvant également être appelée étape de planification d'une trajectoire, est effectuée préalablement au traitement complémentaire, et notamment à tout geste médical sur l'individu. Il convient également de souligner que cette trajectoire est non contraignante, pouvant être suivie ou non par le personnel médical.

Dans des modes particuliers de mise en œuvre de l'invention, le procédé d'évaluation post-traitement comprend également une étape d'aide au suivi de la trajectoire planifiée par un opérateur de l'instrument médical.

Dans des modes particuliers de mise en œuvre de l'invention, la trajectoire planifiée et/ou d'une indication de guidage est affichée en temps réel sur un écran d'un dispositif de réalité augmentée.

L'invention concerne également un dispositif électronique comprenant un processeur et une mémoire informatique stockant des instructions d'un procédé selon l'un quelconque des modes de mise en œuvre précédents.

Un tel dispositif électronique peut être par exemple un dispositif de contrôle, un système de navigation, un dispositif robotisé ou un dispositif de réalité augmentée. Le dispositif de contrôle peut notamment être un ordinateur présent dans la salle d'opération ou un serveur distant.

En d'autres termes, l'invention concerne un dispositif électronique comprenant un processeur et une mémoire informatique stockant des instructions d'un procédé d'évaluation post-traitement d'une ablation d'une partie d'une anatomie d'intérêt d'un individu, l'anatomie d'intérêt comprenant au moins une lésion, la partie de l'anatomie d'intérêt ablatée étant appelée région d'ablation, le processeur étant configuré lors de l'exécution des instructions pour :
- effectuer une acquisition d'une image médicale post-opératoire de l'anatomie d'intérêt de l'individu, comprenant tout ou partie de la région d'ablation ;
- effectuer un recalage de l'image post-opératoire et d'une image médicale de l'anatomie d'intérêt de l'individu, acquise avant le traitement chirurgical, dite image médicale pré-opératoire, l'image médicale pré-opératoire et l'image médicale post-opératoire recalées formant un couple d'images médicales de l'anatomie d'intérêt de l'individu ;
- évaluer un risque de récidive de la lésion de l'anatomie d'intérêt de l'individu par une méthode d'apprentissage automatique, de type réseau de neurones, analysant le couple d'images médicales de l'anatomie d'intérêt de l'individu, ladite méthode d'apprentissage automatique étant préalablement entraînée lors d'une phase dite d'entraînement sur une base de données comportant une pluralité de couples d'images médicales (d'une anatomie d'intérêt identique d'un ensemble de patients, chaque couple d'images médicales de la base de données étant associé à un statut de récidive d'une lésion de l'anatomie d'intérêt dudit patient.

Dans des modes de réalisation particuliers de l'invention, le processeur est configuré pour évaluer le risque de récidive à une échéance prédéterminée suite au traitement, chaque couple d'images médicales de la base de données étant également associé à une date de récidive lorsque le statut de récidive est positif.

Dans des modes de réalisation particuliers de l'invention, le processeur est en outre configuré, après le recalage, pour recadrer tout ou partie des couples d'images de la base de données, autour d'une région d'ablation comprise dans l'image post-opératoire de tout ou partie des couples d'images d'entrainement, le recadrage des images étant effectué selon un cadre commun de dimension prédéterminée, l'ensemble des centres de la région d'ablation des couples d'images recadrés formant une constellation de points distincts à l'intérieur du cadre commun.

Dans des modes de réalisation particuliers de l'invention, pour l'ensemble des couples d'images médicales de la base de données, préalablement recadrées, le processeur est en outre configuré pour diviser la partie du corps de l'individu comprise dans l'image en une pluralité d'unités élémentaires de taille unique, le nombre d'unités élémentaires étant partagé en deux parts quasiment égales entre la partie du corps humain délimitée par la région d'ablation et le reste de la partie du corps de l'individu comprise dans l'image.

Dans des modes de réalisation particuliers de l'invention, la base de données de couples d'images médicales comprend au moins un couple d'images dépourvues de région d'ablation.

Dans des modes de réalisation particuliers de l'invention, le processeur est en outre configuré pour déterminer un masque d'ablation complémentaire lorsque le risque de récidive est supérieur à une valeur seuil prédéterminée.

Dans des modes de réalisation particuliers de l'invention, le processeur est en outre configuré pour détecter la région d'ablation dans l'image post-opératoire de l'anatomie d'intérêt de l'individu lors de la détermination d'un masque d'ablation complémentaire.

Dans des modes de réalisation particuliers de l'invention, le processeur est en outre configuré pour segmenter la région d'ablation dans l'image post-opératoire de l'anatomie d'intérêt de l'individu, lors de la détermination d'un masque d'ablation complémentaire.

Dans des modes de réalisation particuliers de l'invention, le processeur est en outre configuré pour détecter la lésion dans l'image pré-opératoire de l'anatomie d'intérêt de l'individu, lors de la détermination d'un masque d'ablation complémentaire

Dans des modes de réalisation particuliers de l'invention, le processeur est en outre configuré pour segmenter la lésion dans l'image pré-opératoire de l'anatomie d'intérêt de l'individu, lors de la détermination d'un masque d'ablation complémentaire

Dans des modes de réalisation particuliers de l'invention, lors de la détermination d'un masque d'ablation complémentaire, le processeur est en outre configuré pour déterminer la position de la récidive en fonction d'au moins un prédicteur de risque choisi parmi la liste comprenant :
- une marge d'ablation entre la région d'ablation et la liaison,
- une distance entre un centre de masse de la lésion et un centre de masse de la région d'ablation,
- la régularité et de la netteté des bords de la région d'ablation par rapport au tissu sain environnant,
- le rapport entre le volume de la lésion et le volume de la région d'ablation,
- une position de la lésion par rapport au centre de l'anatomie d'intérêt.

Dans des modes de réalisation particuliers de l'invention, les images médicales sont des images tridimensionnelles.

Dans des modes de réalisation particuliers de l'invention, chaque image pré-opératoire est acquise selon une première technique d'acquisition d'image et chaque image post-opératoire est acquise selon une deuxième technique d'acquisition d'image, la première technique et la deuxième technique étant identiques ou distinctes.

### BRÈVE DESCRIPTION DES FIGURES

D'autres avantages, buts et caractéristiques particulières de la présente invention ressortiront de la description non limitative qui suit d'au moins un mode de réalisation particulier des dispositifs et procédés objets de la présente invention, en regard des dessins annexés, dans lesquels :
- la figure 1 est une vue schématique d'une intervention médicale ;
- la figure 2 est une vue synoptique d'un procédé d'évaluation post-traitement de l'intervention médicale de la figure 1 ;
- la figure 3 est un exemple d'image médicale tridimensionnelle dans laquelle une région d'ablation est mise en avant, utilisée au cours du procédé illustré en figure 2 ;
- la figure 4 est un exemple de quatre images médicales comprenant chacune un masque d'ablation délimité manuellement par un opérateur et un masque d'ablation prédit par un réseau de neurones du procédé illustré en figure 2 ;
- la figure 5 est un exemple d'image médicale dans laquelle une segmentation automatique d'une lésion et d'une région d'ablation est effectuée au cours d'une étape du procédé illustré en figure 2 ;
- la figure 6 illustre un exemple d'ablation complémentaire tel qu'éventuellement proposé par le procédé illustré en figure 2.

### DESCRIPTION DÉTAILLÉE DE L'INVENTION

La présente description est donnée à titre non limitatif, chaque caractéristique d'un mode de réalisation pouvant être combinée à toute autre caractéristique de tout autre mode de réalisation de manière avantageuse.

On note, dès à présent, que les figures ne sont pas à l'échelle.

### Exemple d'un mode de mise en œuvre particulier

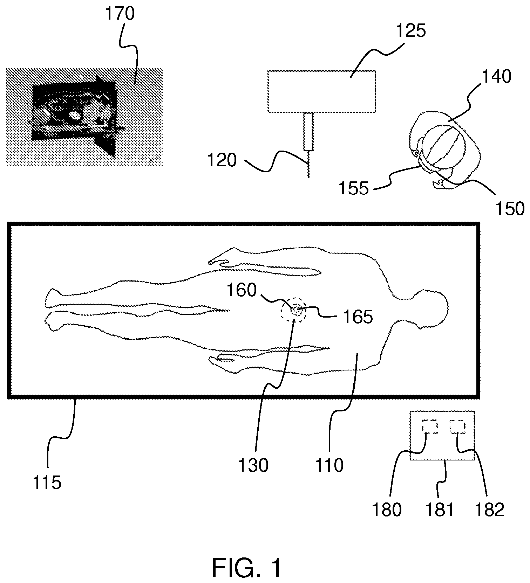

La figure 1 est une vue schématique d'une intervention médicale au cours de laquelle un individu 110 allongé sur une table 115 est traité à l'aide d'un instrument médical 120. Dans le présent exemple non limitatif de l'invention, l'intervention médicale correspond à l'ablation d'une lésion 165 dans une anatomie d'intérêt 130 qui est ici le foie de l'individu 110 par l'intermédiaire de l'instrument médical 120 qui est dans le cas présent une aiguille semi-rigide. L'intervention médicale est ici une procédure percutanée au cours de laquelle le corps de l'individu 110 n'est pas ouvert.

La manipulation de l'instrument médical 120 par un opérateur 140 peut être avantageusement guidée par l'intermédiaire d'un dispositif de guidage qui est dans le présent exemple non limitatif de l'invention un dispositif de réalité augmentée tel qu'un casque 150 porté par l'opérateur 140. L'instrument médical 120 peut également être associé à un dispositif 125 robotisé médical.

Le casque 150 comprend un écran 155 translucide permettant à l'opérateur de voir normalement. Sur l'écran 155, s'affiche en incrustation une image afin d'afficher des repères permettant de guider l'opérateur 140 dans la manipulation de l'instrument médical 120 en vue de traiter par ablation une région 160 dite d'ablation autour de la lésion 165 identifiée dans l'anatomie d'intérêt 130. Les repères peuvent notamment comprendre un masque d'ablation qui a été préalablement estimé sur une image médicale 170 de l'anatomie d'intérêt 130 acquise avant l'opération. L'image médicale 170 sera appelée par la suite image médicale pré-opératoire 170.

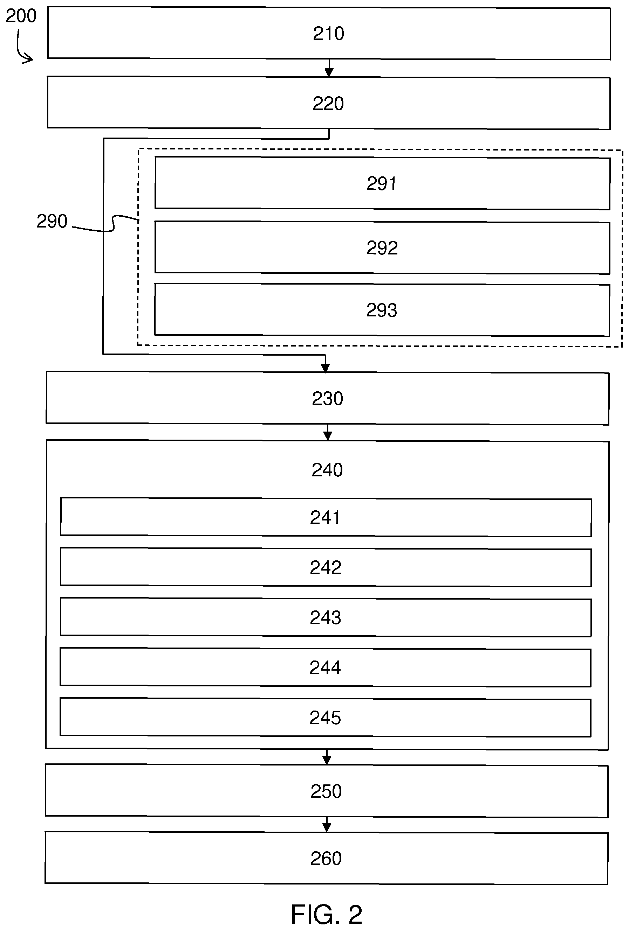

Lorsque l'opération est terminée, une évaluation du traitement opératoire est effectuée par un procédé 200 d'évaluation post-traitement de l'ablation tel qu'illustré sous forme synoptique en figure 2 et dont les instructions sont stockées dans une mémoire informatique 180 d'un dispositif électronique 181 de contrôle relié au casque 155, avec un câble ou par une technologie sans fil. Le procédé 200 d'évaluation post-traitement dont les instructions sont traitées par un processeur informatique 182 du dispositif électronique 181 de contrôle permet notamment de déterminer un risque de récidive associé à la lésion, afin de vérifier si le traitement chirurgical effectué au cours de l'opération est suffisant ou s'il est préférable de poursuivre le traitement en effectuant par exemple une ablation complémentaire.

Il convient de souligner que le dispositif électronique 181 peut être avantageusement intégré dans le casque 150.

Le procédé 200 d'évaluation post-traitement comprend une première étape 210 d'acquisition d'une image médicale post-opératoire de l'anatomie d'intérêt 130.

Il convient de souligner que les images médicales pré-opératoire et post-opératoire sont de préférence acquises par tomodensitométrie. Alternativement elles peuvent être acquises par un appareil d'imagerie par résonance magnétique.

Préférentiellement, la technique utilisée pour acquérir l'image médicale pré-opératoire et l'image médicale post-opératoire est similaire, voire identique.

En d'autres termes, la technique utilisée pour acquérir les images médicales pré-opératoires et post-opératoires est avantageusement identique à celle utilisée pour acquérir les images médicales de la base de données d'entrainement de la méthode d'apprentissage automatique.

Toutefois, la technique utilisée pour acquérir l'image médicale post-opératoire peut être distincte de la technique utilisée pour acquérir l'image médicale pré-opératoire.

Dans ce cas, les techniques utilisées pour acquérir les images médicales pré-opératoires et post-opératoires sont avantageusement identiques à celles utilisées pour acquérir les images médicales de la base de données d'entrainement de la méthode d'apprentissage automatique.

Les images médicales pré-opératoire et post-opératoire sont avantageusement dans le présent exemple non limitatif de l'invention des images acquises en trois dimensions. En pratique, chaque image médicale acquise en trois dimensions correspond généralement à une collection d'images médicales en deux dimensions correspondant chacune à une coupe de l'anatomie d'intérêt 130, effectuée à intervalles réguliers selon un axe prédéterminé. Une représentation tridimensionnelle de l'anatomie d'intérêt peut être reconstruite à partir de cette collection d'images médicales bidimensionnelles. On entendra ainsi par image en trois dimensions aussi bien une collection d'images médicales qu'une représentation tridimensionnelle. On entendra par voxel une unité élémentaire relative à la résolution de l'image en trois dimensions.

Alternativement, les images médicales pré-opératoire et post-opératoire sont acquises chacune en deux dimensions. L'unité élémentaire relative à la résolution de l'image en deux dimensions est alors couramment appelé pixel.

Les images médicales pré- et post-opératoire sont des images comprenant l'anatomie d'intérêt en entier ou recadrées autour de la région d'ablation selon un cadre prédéfini. Sur une image en trois dimensions, le cadre entourant la région d'ablation correspond à un cube. Tandis que sur une image en deux dimensions, le cadre correspond à un carré.

Le cadre entourant la région d'ablation, également connu sous le terme anglais *« bounding box* », peut être généré automatiquement autour de la région d'ablation suite à une action de l'opérateur. Une telle action peut par exemple correspondre au fait que l'opérateur indique un point dans l'image médicale post-opératoire appartenant à la région d'ablation et le cadre est généré autour de ce point. A titre d'exemple, dans le cadre d'un traitement d'ablation mini-invasive sur des lésions de petites tailles, c'est-à-dire par exemple sur des lésions de l'ordre de 5 cm +/- 10% de diamètre maximum, voire de préférence de l'ordre de 3 cm +/- 10% de diamètre maximum, chaque arête du cube ou chaque côté du carré mesure entre 5 et 10 cm.

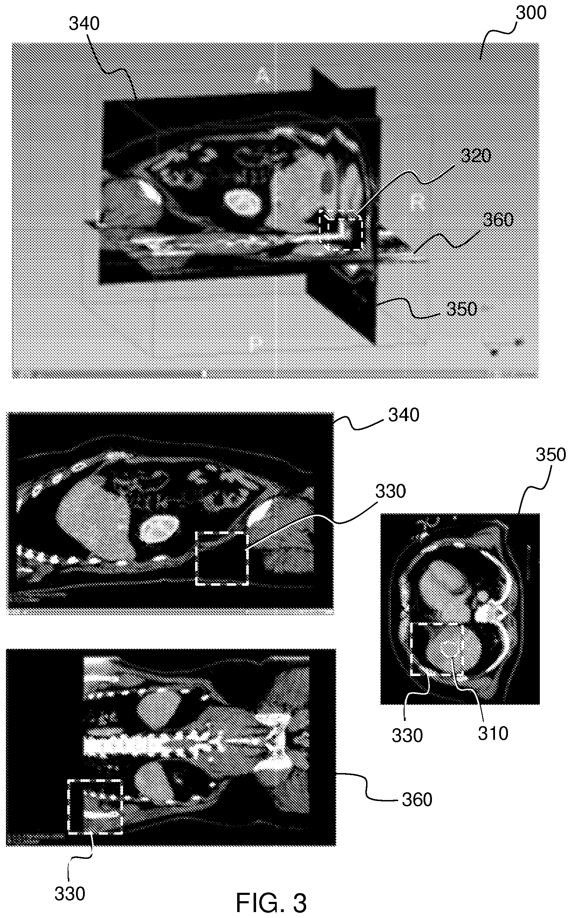

La figure 3 est une illustration d'une image médicale 300 en trois dimensions dans laquelle une région d'ablation 310 est entourée par un cadre 320. Le cadre 320 est cubique et correspond à des carrés 330 sur les vues en coupe 340, 350 et 360, respectivement selon la vue sagittale, selon la vue axiale et selon la vue coronale.

L'image médicale post-opératoire est recalée avec l'image médicale pré-opératoire 170 au cours d'une deuxième étape 220 du procédé 200 illustré en figure 2. Le recalage permettant de retrouver les correspondances entre des points anatomiques des deux images médicales est effectué par une méthode connue de l'homme du métier. Le recalage peut être effectué de manière rigide, c'est-à-dire que tous les points des images sont transformés de la même manière, ou de manière non rigide, c'est-à-dire que chaque point des images peut avoir une transformation spécifique.

Les deux images pré-opératoire et post-opératoire, recalées, forment un couple d'images médicales de l'anatomie d'intérêt 130 de l'individu 110.

Le couple d'images médicales de l'anatomie d'intérêt 130 est ensuite analysé par un réseau de neurones, qui est une méthode d'apprentissage automatique, au cours d'une troisième étape 230 afin d'évaluer automatiquement un risque de récidive associé au traitement par ablation.

À cet effet, le réseau de neurones a préalablement été entraîné sur une base de données de couples d'images médicales d'une anatomie d'intérêt identique, ici par conséquent un foie, d'un ensemble de patients au cours d'une phase préalable 290 d'entraînement. Chaque couple d'images médicales comprend une image pré-opératoire et une image post-opératoire d'une anatomie d'intérêt ayant une fonction identique à celle de l'anatomie d'intérêt 130.

Avantageusement, le couple d'images médicales de l'anatomie d'intérêt 130 de l'individu 110 sont acquises dans la même modalité que celle des images médicales des couples de la base de données d'entraînement du réseau de neurones. En d'autres termes, les images médicales pré-opératoires sont acquises selon une première technique d'imagerie médicale et les images médicales post-opératoires sont acquises selon une deuxième technique d'imagerie médicale qui peut être identique ou différente de la première technique d'imagerie médicale. L'utilisation d'une technique identique, éventuellement avec des paramètres différents, pour le même type d'image médicale, c'est-à-dire pour les images médicales pré-opératoires et/ou pour les images médicales post-opératoires, permet d'améliorer les résultats obtenus par le réseau de neurones en réduisant les biais associés à des techniques d'imagerie médicale distinctes.

Lorsque les images médicales de l'anatomie d'intérêt 130 de l'individu 110 sont recadrées, les dimensions du cube ou du carré de ces images médicales sont avantageusement identiques à celles des cubes ou carrés utilisés pour entraîner le réseau de neurones.

Afin d'entraîner le réseau de neurones, chaque couple d'images médicales de la base de données est associé à un statut de récidive d'une lésion du foie de la personne, le statut indiquant si une récidive a eu lieu ou non, éventuellement associé à la date de récidive. Il convient de souligner que chacun de ces couples d'images médicales provient généralement d'une personne distincte.

La phase 290 d'entraînement du réseau de neurones s'effectue généralement en plusieurs étapes :
- une étape 291 d'entraînement ;
- une étape 292 de validation ;
- une étape 293 de test.

La base de données de couples d'images médicales est ainsi partitionnée en trois bases de données comprenant des couples d'images médicales distincts. Les trois bases de données sont appelées respectivement base d'entrainement, base de validation et base de test. Dans le présent exemple non limitatif de l'invention, 60 à 98 % des couples d'images médicales de la base de données d'images médicales sont regroupées dans la base d'entraînement, 1 à 20 % dans la base de validation et 1 à 20 % dans la base de test. Les pourcentages, fonctions généralement du nombre d'images de la base de données d'images médicales, sont donnés ici à titre indicatif.

Les deux premières étapes 291 et 292 de la phase 290 d'entrainement du réseau de neurones sont des étapes principales pouvant être répétées plusieurs fois. La troisième étape de test est quant à elle optionnelle.

Lors de la première étape 291 de la phase 290 d'entrainement, un poids W et un biais b pour chaque neurone du réseau de neurones sont déterminés à partir des couples d'images médicales de la base d'entrainement.

Il convient de souligner que la base d'entrainement peut avantageusement comprendre des couples d'images sans région d'ablation.

En outre, il peut être préférable que l'ensemble des images de la base de données comprenne autant de voxels appartenant à la région d'ablation que de voxels appartenant à une région de non-ablation. Cette proportion est calculée à partir de la classification des voxels déterminée manuellement par des opérateurs.

En d'autres termes, pour l'ensemble des couples d'images médicales recadrées de la base de données, la partie du corps de l'individu comprise dans l'image est divisée en une pluralité d'unités élémentaires de taille unique, le nombre d'unités élémentaires étant partagé en deux parts quasiment égales entre la partie du corps humain délimitée par la région d'ablation et le reste de la partie du corps de l'individu comprise dans l'image.

On entendra par parts quasiment égales lorsque les deux ensembles d'unités élémentaires sont constitués du même nombre d'unités élémentaires ou lorsque la différence de nombre d'unités élémentaires de chacun des deux ensembles est par exemple inférieure à 5 % du nombre d'unités élémentaires des deux ensembles.

Par ailleurs, la position de la région d'ablation d'une partie des couples d'images médicales recalées peut avantageusement être décalée de manière aléatoire par rapport au centre de l'image de manière aléatoire, afin d'éviter l'introduction d'un biais dans le réseau de neurones qui apprendrait que la région d'ablation est principalement une région au centre du cadre. Il existe en effet des images médicales mal cadrées par l'opérateur autour de la région d'ablation. Une variable aléatoire bornée est par conséquent avantageusement ajoutée à la position des cadres pour limiter ce biais de positionnement de la lésion au centre du cadre.

La deuxième étape 292 de la phase 290 d'entrainement permet de valider le poids W et le biais b déterminés préalablement pour chaque neurone du réseau de neurones, à partir des couples d'images médicales de la base de validation, afin de vérifier les résultats du réseau de neurones, notamment l'erreur de prédiction, c'est-à-dire en comparant pour chaque couple d'images médicales de la base de données de validation , c'est-à-dire le risque de récidive obtenu avec le réseau de neurones, avec respectivement le statut de récidive associé au couple d'images médicales.

Au cas où l'erreur de prédiction serait trop importante à l'issue de cette deuxième étape, les deux étapes d'entrainement 291 et de validation 292 sont mises en œuvre à nouveau pour réentraîner le réseau de neurones en réutilisant les mêmes couples d'images médicales, afin d'affiner les valeurs des poids W et des biais b de chaque neurone.

Alternativement, au cours du réentrainement du réseau de neurones, la première étape 291 utilise un rééchantillonnage des couples d'images médicales en considérant pour l'entrainement les couples d'images médicales de la base de données d'entrainement et une partie des couples d'images médicales de la base de validation. Le reste des couples d'images médicales de la base de validation est ensuite utilisé pour valider les poids W et les biais b obtenus à l'issu de la première étape du réentrainement.

Il convient de souligner que le réseau de neurones peut être réentraîné autant de fois que nécessaire jusqu'à que l'erreur de prédiction soit acceptable, c'est-à-dire soit inférieure à une valeur prédéterminée.

Lorsque les deux étapes 291 et 292 de la phase 290 d'entrainement sont mises en œuvre au moins une fois, les performances finales du réseau de neurones peuvent être testées au cours d'une éventuelle troisième étape 293 de test avec les couples d'images médicales de la base de test. Ces couples d'images médicales, distinctes des couples d'images médicales des bases d'entrainement et de validation, permettent de vérifier que le réseau de neurones tel que configuré avec les paramètres W et b pour chaque neurone permet de prédire avec une bonne précision le risque de récidive dans toutes les situations auxquelles le réseau de neurones est susceptible d'être confronté. Toutefois, à la différence de l'étape 292 de validation, cette éventuelle troisième étape 293 de test n'aboutit pas à un nouveau cycle d'entrainement du réseau de neurones.

Il convient de souligner que les images utilisées lors de l'étape 293 de test sont généralement soigneusement sélectionnées afin de couvrir différentes positions et tailles de la région d'ablation dans l'anatomie d'intérêt afin de tester au mieux les capacités de prédiction du réseau de neurones.

La prédiction d'un risque de récidive associé au traitement chirurgical est obtenue directement par le réseau de neurones qui a été préalablement entraîné sur une base de données de couples d'images médicales associé chacun à un statut de récidive.

Le statut de récidive associé est par exemple égal à 0 lorsqu'il n'y pas eu de récidive de la lésion dans un délai donné suite à l'ablation (statut négatif) ou à 1 lorsqu'il y a eu une récidive de la lésion dans un délai donné suite à l'ablation (statut positif). De préférence, le délai associé à l'estimation du statut de récidive est de 6 mois. Il est cependant possible d'estimer le statut de récidive à différentes échéances suite à l'ablation en associant une date de récidive lorsque le statut de récidive est positif. Par exemple, le réseau de neurones peut être également entrainé pour prédire les statuts de récidive associés à un couple d'images médicales tous les 6 mois suite à l'ablation ou à 1 mois, 3 mois, 6 mois, 1 an, 2 ans, 5 ans ou 10 ans suite à l'ablation.

La valeur du risque de récidive de la lésion prend généralement la forme d'une probabilité entre 0 et 1.

Dans le cas où la valeur du risque de récidive est supérieure à une valeur seuil prédéterminée, c'est-à-dire que la probabilité qu'une récidive intervienne éventuellement dans une échéance donnée, un traitement complémentaire peut être proposé par le procédé 200 d'évaluation post-traitement en estimant un masque d'ablation complémentaire estimé au cours d'une quatrième étape 240 du procédé 200 d'évaluation post-traitement. Ce traitement complémentaire, non contraignant, correspond par exemple à une ablation complémentaire dans une région où la marge d'ablation est inférieure à une valeur donnée, par exemple cinq millimètres.

L'étape 240 de génération d'un masque d'ablation complémentaire comprend généralement cinq sous-étapes numérotées 241 à 245 sur la figure 2.

Au cours de la sous-étape 241, la lésion 165 est détectée dans l'image médicale pré-opératoire de l'anatomie d'intérêt 130 de l'individu 110. Cette détection peut être effectuée automatiquement ou manuellement par un opérateur.

Lors de la sous-étape 242, un masque d'ablation autour d'au moins une lésion de l'anatomie d'intérêt, est généré par un deuxième réseau de neurones à partir de l'apprentissage d'images médicales d'une deuxième base de données. Dans cette deuxième base de données, les images médicales comprennent pour la majorité d'entre elles une région d'ablation préalablement détectée et segmentée sur un patient présentant une lésion dans le foie.

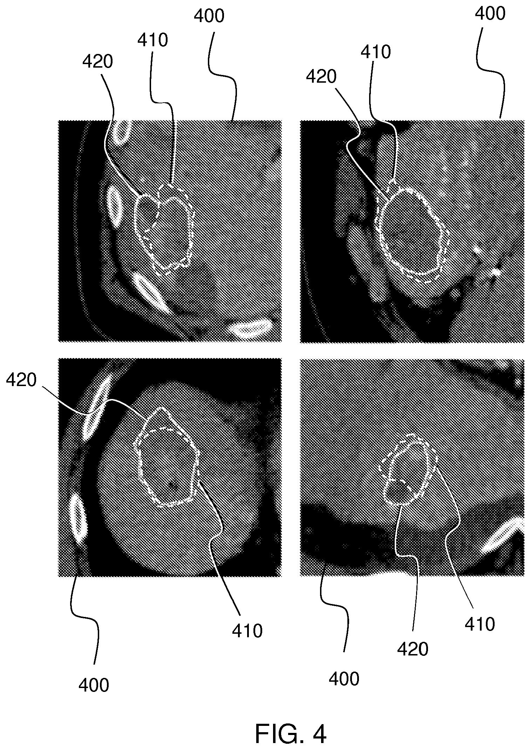

La figure 4 illustre quatre images médicales 400 de cette deuxième base de données. Chaque image médicale 400 a été annotée manuellement par un opérateur qui a délimité une région 410 d'ablation. Le réseau de neurones a généré un masque 420 d'ablation.

Il convient de souligner que pour chaque image médicale de la deuxième base de données, la région d'ablation a été avantageusement déterminée manuellement par au moins deux opérateurs, afin d'augmenter la pertinence de l'apprentissage et par conséquent des résultats d'analyse obtenus par le deuxième réseau de neurones. En effet, il peut être difficile pour un opérateur de délimiter une région d'ablation, notamment quand le contraste dans l'image est faible comme dans les images 400 de la figure 4. L'utilisation de plusieurs opérateurs annotant les images médicales permet donc d'améliorer l'identification de la région d'ablation. La région d'ablation associée au couple d'images médicales pré- et post-opératoires recalées correspond ainsi dans le présent exemple non limitatif de l'invention à l'union des régions d'ablation proposées par les opérateurs. A titre d'exemple alternatif, la région d'ablation associée à une image médicale peut correspondre à l'intersection, à un consensus ou à une adjudication des régions d'ablation proposées par les opérateurs. Le réseau de neurones est en outre entrainé à classer les voxels d'une image médicale en région d'ablation ou de non-ablation.

Alternativement, l'apprentissage peut être effectué en utilisant un seul annotateur expert délimitant les régions d'ablation dans les images médicales. L'expérience de l'opérateur est alors importante afin que le deuxième réseau de neurones puisse aboutir à des régions d'ablation bien délimitées.

Il convient également de souligner que la deuxième base de données peut comprendre les mêmes avantages que la première base de données afin de limiter les biais d'apprentissage du deuxième réseau de neurones, à savoir qu'elle peut comprendre des images sans région d'ablation, que la position des régions d'ablation ne sont pas systématiquement au centre de l'image et que l'ensemble des images de la base de données comprend autant de voxels dans la région d'ablation que dans la région de non-ablation de la partie d'anatomie visible dans l'image.

A partir de l'image médicale post-opératoire de l'anatomie d'intérêt 130 de l'individu, le deuxième réseau de neurones classe chaque voxel en région d'ablation ou de non ablation. Cette prédiction peut prendre la forme d'un masque d'ablation superposé au couple d'images médicales de l'anatomie d'intérêt 130. Le masque d'ablation est généralement recalé sur les voxels appartenant à la région d'ablation prédite par le deuxième réseau de neurones. Il convient de souligner que le masque d'ablation est généralement délimité par une surface ou par un contour dans le cadre d'une image bidimensionnelle.

Au cours de la sous-étape 243, une segmentation de la lésion 165 est effectuée automatiquement sur l'image médicale pré-opératoire de l'anatomie d'intérêt 130 de l'individu 110. Alternativement, la segmentation est effectuée manuellement par un opérateur.

La lésion est segmentée automatiquement par des méthodes connues de l'homme du métier. Par exemple, la segmentation est effectuée par une méthode basée sur l'histogramme de l'image, telle que par exemple la méthode d'Otsu, ou par une méthode d'apprentissage profond, plus couramment appelé par le terme anglais *« deep learning ».*

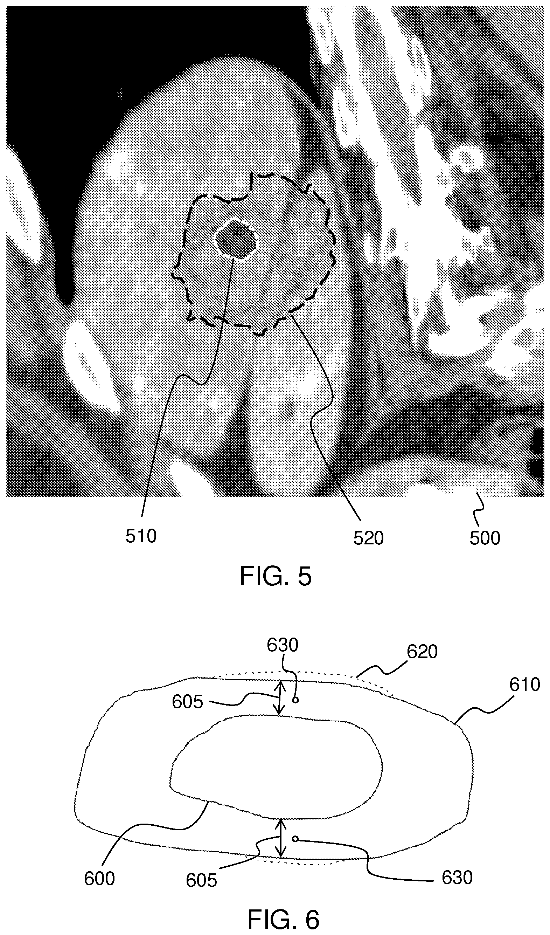

Cette sous-étape 243 de segmentation est illustrée par la figure 5 qui présente une image médicale 500 pré-opératoire dans laquelle une segmentation automatique basée sur une méthode d'apprentissage profond est effectuée pour déterminer l'emplacement tridimensionnel de la lésion 510 et de la région d'ablation 520. Un résultat équivalent peut être obtenu par un troisième réseau de neurones distinct des deux réseaux de neurones utilisé précédemment.

Une marge d'ablation est ensuite déterminée entre la segmentation de la lésion et le masque d'ablation préalablement établis, au cours de la sous-étape 244. La marge d'ablation correspond au minimum de marge, c'est-à-dire au minimum de distance, relevée entre la segmentation de la lésion et le masque d'ablation. En d'autres termes, la marge d'ablation correspond à la plus petite distance calculée entre un point de la lésion et un point de la région d'ablation et se calcule pour tous les points de la lésion.

La détermination des marges d'ablation permet de s'assurer que la région d'ablation recouvre bien la lésion.

La figure 6 illustre un exemple d'ablation complémentaire suite à un traitement d'ablation d'une lésion 600 avec risque de récidive. Le procédé 200 d'évaluation post-traitement a identifié des régions dans lesquelles les marges d'ablation 605 étaient insuffisantes entre la lésion 600 et la région d'ablation 610, et a généré un masque 620 d'ablation complémentaire. Il convient de souligner que la génération du masque 620 d'ablation complémentaire est effectuée en tentant de limiter au maximum la zone d'ablation. En outre, des points cibles 630 à atteindre par une aiguille d'ablation peuvent alors être définis dans le masque 620.

En outre, une prédiction de la position de la récidive peut être évaluée au cours de la sous-étape 245 en comparant la marge d'ablation calculée avec des valeurs de référence des marges d'ablations associées à un statut de récidive, stockées dans une base de données. Par exemple, le risque de récidive soit considéré comme faible, voire nul lorsque les marges d'ablation sont supérieures ou égales à 5 mm.

En complément ou de façon alternative, d'autres prédicteurs du risque de récidive que les marges d'ablation peuvent être utilisés. La position de la récidive peut être estimée en pondérant tout ou partie ces différents prédicteurs. A titre d'exemple, les prédicteurs du risque de récidive peuvent être :
- une distance entre la surface de la lésion, ou une partie de la surface de la lésion, et la région d'ablation ;
- une distance entre les centres de masse de la lésion et les centres de masse de la région d'ablation ;
- une distance entre la surface de la lésion, ou une partie de la surface de la lésion et la région d'ablation et la distance entre les centres de masse de la lésion et les centres de masse de la région d'ablation en tenant compte de la proximité de la capsule de l'anatomie d'intérêt, notamment dans le cas des lésions sous-capsulaires ;
- une régularité des bords de la région d'ablation par rapport au tissu sain environnant ;
- une netteté des bords de la région d'ablation par rapport au tissu sain environnant ;
- un rapport entre le volume de la lésion et le volume de la région d'ablation ;
- une position de la lésion dans l'anatomie d'intérêt.

La valeur de référence du centre de masse dépend des marges d'ablation. Si les marges d'ablation sont égales à 10 mm et que la valeur de référence des marges d'ablation est de 5 mm, la distance entre les centres de masse de la lésion et les centres de masse de la région d'ablation doit être inférieure ou égale à 5 mm.

Le procédé 200 peut également comprendre une étape 250 de planification d'une trajectoire à suivre par l'instrument médical 120 associée soit au masque d'ablation ou au masque d'ablation complémentaire, afin de guider l'opérateur lors de la manipulation de l'instrument médical 120 au cours d'une étape 260 de guidage de l'instrument médical 120 selon la trajectoire planifiée.

Un exemple de méthode de planification est décrit dans la demande de brevet français n°1914780 intitulée *« Méthode de planification automatique d'une trajectoire pour une intervention médicale ».*

Il convient de souligner que le guidage est dans le présent exemple non limitatif de l'invention du type visuel en affichant la trajectoire planifiée et/ou d'une indication de guidage sur l'écran 155 du casque 150.

Alternativement, le guidage de l'instrument médical 120 peut être effectué par l'intermédiaire d'un système de navigation fournissant des informations de position et d'orientation de l'instrument médical 120. Il peut s'agir d'un guidage mécanique par l'intermédiaire d'un dispositif robotisé couplé à un tel système de navigation.

Il convient de souligner que les étapes 230 à 260 peuvent être répétées jusqu'à ce que le risque de récidive soit nul ou quasiment nul, ou jusqu'à ce que les marges d'ablation soient suffisantes.

## Revendications

1. Procédé (200) d'évaluation post-traitement d'une ablation d'une partie d'une anatomie d'intérêt (130) d'un individu (110), l'anatomie d'intérêt comprenant au moins une lésion (165, 510, 600), la partie de l'anatomie d'intérêt ablatée étant appelée région d'ablation (160), **caractérisé en ce que** le procédé d'évaluation post-traitement comprend des étapes de :
• acquisition (210) d'une image médicale post-opératoire de l'anatomie d'intérêt de l'individu, comprenant tout ou partie de la région d'ablation ;
• recalage (220) de l'image post-opératoire et d'une image médicale (170, 500) de l'anatomie d'intérêt de l'individu, acquise avant le traitement chirurgical, dite image médicale pré-opératoire, l'image médicale pré-opératoire et l'image médicale post-opératoire recalées formant un couple d'images médicales de l'anatomie d'intérêt de l'individu ;
• évaluation d'un risque de récidive de la lésion de l'anatomie d'intérêt de l'individu par une méthode d'apprentissage automatique, de type réseau de neurones, analysant le couple d'images médicales de l'anatomie d'intérêt de l'individu, ladite méthode d'apprentissage automatique étant préalablement entraînée lors d'une phase (290) dite d'entraînement sur une base de données comportant une pluralité de couples d'images médicales (300, 400) d'une anatomie d'intérêt identique d'un ensemble de patients, chaque couple d'images médicales de la base de données étant associé à un statut de récidive d'une lésion de l'anatomie d'intérêt dudit patient.

2. Procédé d'évaluation post-traitement selon la revendication 1, dans lequel le risque de récidive est évalué à une échéance prédéterminée suite au traitement, chaque couple d'images médicales de la base de données étant également associé à une date de récidive lorsque le statut de récidive est positif.

3. Procédé d'évaluation post-traitement selon l'une quelconque des revendications précédentes, dans lequel tout ou partie des couples d'images de la base de données sont recadrés, après recalage, autour d'une région d'ablation comprise dans l'image post-opératoire de tout ou partie des couples d'images d'entrainement, le recadrage des images étant effectué selon un cadre commun de dimension prédéterminée, l'ensemble des centres de la région d'ablation des couples d'images recadrés formant une constellation de points distincts à l'intérieur du cadre commun.

4. Procédé d'évaluation post-traitement selon la revendication précédente, dans lequel pour l'ensemble des couples d'images médicales de la base de données, préalablement recadrées, la partie du corps de l'individu comprise dans l'image est divisée en une pluralité d'unités élémentaires de taille unique, le nombre d'unités élémentaires étant partagé en deux parts quasiment égales entre la partie du corps humain délimitée par la région d'ablation et le reste de la partie du corps de l'individu comprise dans l'image.

5. Procédé d'évaluation post-traitement selon l'une quelconque des revendications précédentes, dans lequel la base de données de couples d'images médicales comprend au moins un couple d'images dépourvues de région d'ablation.

6. Procédé d'évaluation post-traitement selon l'une quelconque des revendications précédentes, comprenant également une étape de détermination d'un masque d'ablation complémentaire lorsque le risque de récidive est supérieur à une valeur seuil prédéterminée.

7. Procédé d'évaluation post-traitement selon la revendication 6, dans lequel l'étape de détermination d'un masque d'ablation complémentaire comprend une sous-étape de détection de la région d'ablation dans l'image post-opératoire de l'anatomie d'intérêt de l'individu.

8. Procédé d'évaluation post-traitement selon l'une quelconque des revendications 6 à 7, dans lequel l'étape de détermination d'un masque d'ablation complémentaire comprend une sous-étape de segmentation de la région d'ablation dans l'image post-opératoire de l'anatomie d'intérêt de l'individu.

9. Procédé d'évaluation post-traitement selon l'une quelconque des revendications 6 à 8, dans lequel l'étape de détermination d'un masque d'ablation complémentaire comprend une sous-étape de détection de la lésion dans l'image pré-opératoire de l'anatomie d'intérêt de l'individu.

10. Procédé d'évaluation post-traitement selon l'une quelconque des revendications 6 à 9, dans lequel l'étape de détermination d'un masque d'ablation complémentaire comprend une sous-étape de segmentation de la lésion dans l'image pré-opératoire de l'anatomie d'intérêt de l'individu.

11. Procédé d'évaluation post-traitement selon l'une quelconque des revendications 6 à 9, dans lequel l'étape de détermination d'un masque d'ablation complémentaire comprend une sous-étape de détermination de la position de la récidive en fonction d'au moins un prédicteur de risque choisi parmi la liste comprenant :
• une marge d'ablation entre la région d'ablation et la lésion,
• une distance entre un centre de masse de la lésion et un centre de masse de la région d'ablation,
• la régularité et de la netteté des bords de la région d'ablation par rapport au tissu sain environnant,
• le rapport entre le volume de la lésion et le volume de la région d'ablation,
• une position de la lésion par rapport au centre de l'anatomie d'intérêt.

12. Procédé d'évaluation post-traitement selon l'une quelconque des revendications précédentes, dans lequel les images médicales sont des images tridimensionnelles.

13. Procédé d'évaluation post-traitement selon l'une quelconque des revendications précédentes, dans lequel chaque image pré-opératoire est acquise selon une première technique d'acquisition d'image et chaque image post-opératoire est acquise selon une deuxième technique d'acquisition d'image, la première technique et la deuxième technique étant identiques ou distinctes.

14. Dispositif (150) électronique comprenant un processeur et une mémoire informatique stockant des instructions d'un procédé d'évaluation post-traitement selon l'une quelconque des revendications précédentes.

15. Dispositif électronique selon la revendication précédente, pouvant être un dispositif de contrôle, un système de navigation, un dispositif robotisé ou un dispositif de réalité augmentée.

## Patentansprüche

1. Verfahren (200) zur Nachbehandlungsbewertung einer Ablation eines Teils einer Anatomie (130) von Interesse einer Person (110), wobei die Anatomie von Interesse mindestens eine Läsion (165, 510, 600) umfasst, wobei der abladierte Teil der Anatomie von Interesse als Ablationsbereich (160) bezeichnet wird, **dadurch gekennzeichnet, dass** das Verfahren zur Nachbehandlungsbewertung folgende Schritte umfasst:
• Erfassen (210) eines postoperativen medizinischen Bildes der Anatomie von Interesse der Person, das den gesamten oder einen Teil des Ablationsbereichs umfasst;
• Registrieren (220) des postoperativen Bildes und eines medizinischen Bildes (170, 500) der Anatomie von Interesse der Person, das vor der chirurgischen Behandlung erfasst wurde, des präoperativen medizinischen Bildes, wobei das registrierte präoperative medizinische Bild und das registrierte postoperative medizinische Bild ein medizinisches Bildpaar der Anatomie von Interesse der Person bilden;
• Bewerten eines Risikos eines Rezidivs der Läsion der Anatomie von Interesse der Person durch ein automatisches Lernverfahren, vom Typ Neuronennetzwerk, durch Analyse des medizinischen Bildpaars der Anatomie von Interesse der Person, wobei das automatische Lernverfahren zuvor während einer Trainingsphase (290) auf einer Datenbank mit einer Vielzahl von medizinischen Bildpaaren (300, 400) einer identischen Anatomie von Interesse einer Gruppe von Patienten trainiert wird, wobei jedes medizinische Bildpaar aus der Datenbank mit einem Rezidivstatus einer Läsion der Anatomie von Interesse des Patienten verbunden ist.

2. Verfahren zur Nachbehandlungsbewertung nach Anspruch 1, wobei das Rezidivrisiko zu einem vorbestimmten Zeitpunkt nach der Behandlung bewertet wird, wobei jedes medizinische Bildpaar aus der Datenbank auch mit einem Rezidivdatum verbunden ist, wenn der Rezidivstatus positiv ist.

3. Verfahren zur Nachbehandlungsbewertung nach einem der vorhergehenden Ansprüche, wobei alle oder ein Teil der Bildpaare der Datenbank nach der Registrierung um einen Ablationsbereich, der in dem postoperativen Bild aller oder eines Teils der Trainingsbildpaare enthalten ist, zugeschnitten werden, wobei das Zuschneiden der Bilder nach einem gemeinsamen Rahmen vorbestimmter Größe durchgeführt wird, wobei alle Zentren des Ablationsbereichs der zugeschnittenen Bildpaare eine Konstellation von verschiedene Punkten innerhalb des gemeinsamen Rahmens bilden.

4. Verfahren zur Nachbehandlungsbewertung nach dem vorhergehenden Anspruch, wobei für alle zuvor zugeschnittenen medizinischen Bildpaare aus der Datenbank der in dem Bild umfasste Körperteil der Person in eine Vielzahl elementarer Einheiten einheitlicher Größe aufgeteilt ist, wobei die Anzahl der elementaren Einheiten in nahezu gleiche Teile zwischen dem durch den Ablationsbereich begrenzten Teil des menschlichen Körpers und dem restlichen Teil des Körpers der Person aufgeteilt wird, der in dem Bild umfasst ist.

5. Verfahren zur Nachbehandlungsbewertung nach einem der vorhergehenden Ansprüche, wobei die Datenbank mit medizinischen Bildpaaren mindestens ein Bildpaar ohne Ablationsbereich umfasst.

6. Verfahren zur Nachbehandlungsbewertung nach einem der vorhergehenden Ansprüche, ferner umfassend einen Schritt zum Bestimmen einer zusätzlichen Ablationsmaske, wenn das Risiko eines Rezidivs über einem vorbestimmten Schwellenwert liegt.

7. Verfahren zur Nachbehandlungsbewertung nach Anspruch 6, wobei der Schritt zum Bestimmen einer zusätzlichen Ablationsmaske einen Unterschritt zum Erkennen des Ablationsbereichs in dem postoperativen Bild der Anatomie von Interesse der Person umfasst.

8. Verfahren zur Nachbehandlungsbewertung nach einem der Ansprüche 6 bis 7, wobei der Schritt zum Bestimmen einer zusätzlichen Ablationsmaske einen Unterschritt zum Segmentieren des Ablationsbereichs in dem postoperativen Bild der Anatomie von Interesse der Person umfasst.

9. Verfahren zur Nachbehandlungsbewertung nach einem der Ansprüche 6 bis 8, wobei der Schritt zum Bestimmen einer zusätzlichen Ablationsmaske einen Unterschritt zum Erkennen der Läsion in dem präoperativen Bild der Anatomie von Interesse der Person umfasst.

10. Verfahren zur Nachbehandlungsbewertung nach einem der Ansprüche 6 bis 9, wobei der Schritt des Bestimmens einer zusätzlichen Ablationsmaske einen Unterschritt des Segmentierens der Läsion in dem präoperativen Bild der Anatomie von Interesse der Person umfasst.

11. Verfahren zur Nachbehandlungsbewertung nach einem der Ansprüche 6 bis 9, wobei der Schritt zum Bestimmen einer zusätzlichen Ablationsmaske einen Unterschritt zum Bestimmen der Position des Rezidivs basierend auf mindestens einem Risikoprädikator umfasst, der aus der Liste ausgewählt ist, umfassend:
• einen Ablationsrand zwischen dem Ablationsbereich und der Läsion,
• einen Abstand zwischen einem Massenzentrum der Läsion und einem Massenzentrum des Ablationsbereichs,
• die Gleichmäßigkeit und Schärfe der Ränder des Ablationsbereichs im Verhältnis zu dem umgebenden gesunden Gewebe,
• das Verhältnis zwischen dem Volumen der Läsion und dem Volumen des Ablationsbereichs,
• eine Position der Läsion im Verhältnis zu dem Zentrum der Anatomie von Interesse.

12. Verfahren zur Nachbehandlungsbewertung nach einem der vorhergehenden Ansprüche, wobei die medizinischen Bilder dreidimensionale Bilder sind.

13. Verfahren zur Nachbehandlungsbewertung nach einem der vorhergehenden Ansprüche, wobei jedes präoperative Bild nach einem ersten Bilderfassungsverfahren und jedes postoperative Bild nach einem zweiten Bilderfassungsverfahren erfasst wird, wobei das erste Verfahren und das zweite Verfahren identisch oder verschieden sind.

14. Elektronische Vorrichtung (150), die einen Prozessor und einen Computerspeicher zum Speichern von Anweisungen eines Verfahrens zur Nachbehandlungsbewertung nach einem der vorhergehenden Ansprüche umfasst.

15. Elektronische Vorrichtung nach dem vorhergehenden Anspruch, die eine Kontrollvorrichtung, ein Navigationssystem, eine Robotervorrichtung oder eine Augmented-Reality-Vorrichtung sein kann.

## Claims

1. A method (200) for post-treatment evaluating an ablation of a part of an anatomy of interest (130) of an individual (110), the anatomy of interest comprising at least one lesion (165, 510, 600), the part of the anatomy of interest being referred to as the ablation region (160), **characterised in that** the post-treatment evaluation method comprises steps of:
• acquiring (210) a postoperative medical image of the anatomy of interest of the individual, comprising all or part of the ablation region;
• registering (220) the postoperative image and a medical image (170, 500) of the anatomy of interest of the individual, acquired prior to the surgical treatment, so-called preoperative medical image, the registered preoperative medical image and the registered postoperative medical image forming a pair of medical images of the anatomy of interest of the individual;
• evaluating a risk of recurrence of the lesion of the anatomy of interest of the individual by a machine learning method, of the neural network type, analysing the pair of medical images of the anatomy of interest of the individual, said machine learning method being previously trained during a so-called training phase (290) on a database comprising a plurality of pairs of medical images (300, 400) of an identical anatomy of interest of a set of patients, each pair of medical images of the database being associated with a recurrence status of a lesion of the anatomy of interest of said patient.

2. The post-treatment evaluation method according to claim 1, wherein the risk of recurrence is evaluated at a predetermined time point following the treatment, each pair of medical images from the database also being associated with a recurrence date when the recurrence status is positive.

3. The post-treatment evaluation method according to any one of the preceding claims, wherein all or part of the image pairs of the database are reframed, after registration, around an ablation region included in the postoperative image of all or part of the pairs of training images, reframing of the images being performed according to a common frame of a predetermined size, all the centres of the ablation region of the pairs of reframed images forming a constellation of distinct points within the common frame.

4. The post-treatment evaluation method according to the preceding claim, wherein for all the pairs of medical images of the database, previously reframed, the part of the body of the individual included in the image is divided into a plurality of single-sized elementary units, the number of elementary units being split in nearly equal parts between the part of the human body delimited by the ablation region and the rest of the part of the body of the individual included in the image.

5. The post-treatment evaluation method according to any one of the preceding claims, wherein the database of pairs of medical images comprises at least one pair of images free of ablation region.

6. The post-treatment evaluation method according to any one of the preceding claims, also comprising a step of determining a complementary ablation mask when the risk of recurrence is greater than a predetermined threshold value.

7. The post-treatment evaluation method according to claim 6, wherein the step of determining a complementary ablation mask comprises a sub-step of detecting the ablation region in the postoperative image of the anatomy of interest of the individual.

8. The post-treatment evaluation method according to any one of claims 6 to 7, wherein the step of determining a complementary ablation mask comprises a sub-step of segmenting the ablation region in the postoperative image of the anatomy of interest of the individual.

9. The post-treatment evaluation method according to any one of claims 6 to 8, wherein the step of determining a complementary ablation mask comprises a sub-step of detecting the lesion in the preoperative image of the anatomy of interest of the individual.

10. The post-treatment evaluation method according to any one of claims 6 to 9, wherein the step of determining a complementary ablation mask comprises a sub-step of segmenting the lesion in the preoperative image of the anatomy of interest of the individual.

11. The post-treatment evaluation method according to any one of claims 6 to 9, wherein the step of determining a complementary ablation mask comprises a sub-step of determining the position of the recurrence according to at least one risk predictor chosen from the list comprising:
• an ablation margin between the ablation region and the lesion,
• a distance between a centre of mass of the lesion and a centre of mass of the ablation region,
• the regularity and sharpness of the edges of the ablation region relative to the surrounding healthy tissue,
• the ratio of the lesion volume to the ablation region volume,
• a position of the lesion relative to the centre of the anatomy of interest.

12. The post-treatment evaluation method according to any one of the preceding claims, wherein the medical images are three-dimensional images.

13. The post-treatment evaluation method according to any one of the preceding claims, wherein each preoperative image is acquired according to a first image acquisition technique and each postoperative image is acquired according to a second image acquisition technique, the first technique and the second technique being identical or distinct.

14. An electronic device (150) comprising a processor and a computer memory storing instructions of a post-treatment evaluation method according to any one of the preceding claims.

15. The electronic device according to the preceding claim, which may be a control device, a navigation system, a robotic device or an augmented reality device.
